# EUROPEAN PATENT APPLICATION

(11) **EP 4 714 462 A1**
(43) Date of publication of application: **25.03.2026**
(21) Application number: 24807638.2
(22) Date of filing: 17.05.2024
(51) Int. Cl.: A61K 38/10, A61P 27/02

(54) **COMPOSITION COMPRISING GV1001 FOR PREVENTING OR TREATING MACULAR DEGENERATION**

(30) Priority: 17.05.2023 KR 20230063624
(71) Applicant: Gemvax & Kael Co., Ltd., Daejeon 34036 (KR)
(72) Inventor: KIM, Sang Jae, Seoul 06360 (KR)
(74) Representative: Stolmár & Partner Patentanwälte PartG mbB
(86) International application number: PCT/KR2024/095811
(87) International publication number: WO 2024/237768

(57) **Abstract**

The present invention relates to a pharmaceutical composition for prevention or treatment of a macular degeneration, more particularly, a pharmaceutical composition for preventing or treating the macular degeneration, which includes a peptide having the amino acid sequence of SEQ ID NO: 1, so that it exhibits effects of reducing retinal degeneration and is safe to the living body to thus involve less side effects including abnormal response.

## Description

### [Technical Field]

The present invention relates to a composition including GV1001 for preventing or treating macular degeneration.

### [Background Art]

Age-related macular degeneration (AMD) is an ocular disease in which the function of the macula, the central part of the retina where images are formed, is impaired, resulting in gradual loss of vision starting from the center of the visual field and eventually leading to blindness. AMD can be classified into dry AMD, which occurs due to the accumulation of cellular metabolic waste products such as drusen and amyloid beta (Aβ), and wet AMD, which results from the formation of new blood vessels and subsequent hemorrhage or fluid leakage that cause vision loss.

Dry AMD occurs when retinal cells are continuously exposed to stress conditions such as oxidative stress, leading to retinal cell death and degeneration. The death and degeneration of retinal cells loosen the compact retinal layer, and neovascularization induced by secreted cytokines and growth factors causes progression into wet AMD. Therefore, treatment at the dry AMD stage is of critical importance.

In the case of wet AMD, neovascularization induced by vascular endothelial growth factor (VEGF) is known to be a direct cause, and drugs targeting VEGF, such as Eylea^{®}, are currently used as therapeutic agents.

Unlike wet AMD, for which many therapeutic agents have been commercialized, only one FDA-approved drug exists for dry AMD to date. Therefore, the development of a therapeutic agent for dry AMD capable of controlling the early progression of AMD is urgently required.

Accordingly, the inventors, as a result of repeated studies, discovered that age-related macular degeneration can be treated by reducing reactive oxygen species (ROS) and decreasing the degeneration of retinal pigment epithelial (RPE) cells, and thereby completed the present invention.

### [Summary of Invention]

### [Problems to be Solved by Invention]

An object of the present invention is to provide a pharmaceutical composition for preventing or treating macular degeneration.

Another object of the present invention is to provide a pharmaceutical composition for preventing or treating age-related macular degeneration.

Yet another object of the present invention is to provide a kit for preventing or treating macular degeneration.

Still another object of the present invention is to provide a health functional food for preventing or improving macular degeneration.

A further object of the present invention is to provide a health functional food for preventing or improving age-related macular degeneration.

### [Means for Solving Problems]

1. A pharmaceutical composition for use in preventing or treating a macular degeneration, including a peptide having the amino acid sequence of SEQ ID NO: 1.
2. The pharmaceutical composition according to the above 1, wherein the macular degeneration is age-related macular degeneration.
3. The pharmaceutical composition according to the above 1, wherein the pharmaceutical composition prevents or treats the macular degeneration by reducing degeneration of retinal pigment epithelial cells.
4. A kit for use in preventing or treating a macular degeneration, including: the pharmaceutical composition according to any one of the above 1 to 3; and instructions describing a method for preventing or treating the macular degeneration.
5. The kit according to the above 4, wherein the method for preventing or treating a macular degeneration includes administering the pharmaceutical composition to a subject who has the macular degeneration or is at risk of developing the macular degeneration.
6. A health functional food for use in preventing or improving a macular degeneration, including a peptide having the amino acid sequence of SEQ ID NO: 1.
7. The health functional food according to the above 6, wherein the macular degeneration is age-related macular degeneration.
8. The health functional food according to the above 6, wherein the health functional food prevents or improves the macular degeneration by reducing degeneration of retinal pigment epithelial cells.

### [Advantageous effects]

The pharmaceutical composition of the present invention, including the peptide having the amino acid sequence of SEQ ID NO: 1, not only reduced cell degeneration in a model of age-related macular degeneration retinal pigment epithelial cell, but also exhibited inhibitory and/or therapeutic effects on retinal degeneration in a mouse model of age-related macular degeneration. Therefore, the pharmaceutical composition of the present invention is expected to be effective in the treatment, improvement, or prevention of macular degeneration.

The pharmaceutical composition and health functional food of the present invention include GV1001, which has completed toxicity tests in numerous clinical experiments and has been confirmed to be safe from side effects, as an active ingredient, thus is expected to be useful in preventing, improving or treating macular degeneration.

It is expected that the pharmaceutical composition, kit, and health functional food of the present invention will be able to provide economic or medical and social support to patients suffering from macular degeneration and their families in the future.

The present invention is expected to contribute to understanding the pathophysiology of macular degeneration by elucidating the mechanism of the pharmacological effect of GV1001 on macular degeneration, and to serve as a driving force for the development of new therapeutic agents in the future.

### [Brief Description of Drawings]

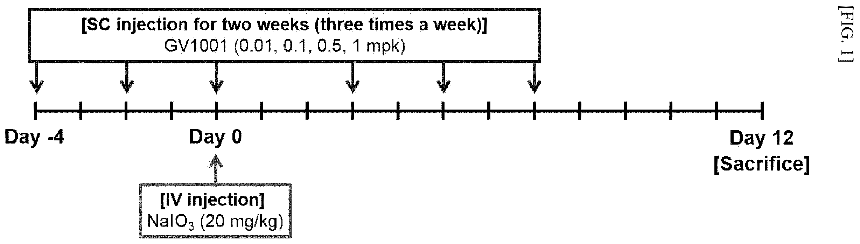

FIG. 1 shows the GV1001 administration time and experimental schedule for the GV1001 pre-treatment animal experimental group used in the GV1001 short-term administration effect evaluation test.

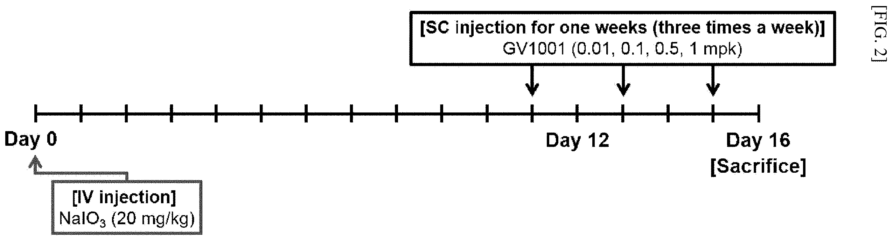

FIG. 2 shows the GV1001 administration time and experimental schedule for the GV1001 post-treatment animal experimental group used in the GV1001 short-term administration effect evaluation test.

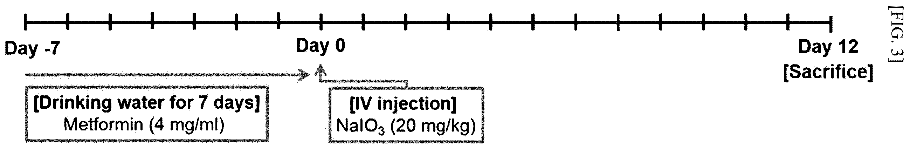

FIG. 3 shows the administration time and experimental schedule of the positive control drug used in the GV1001 short-term administration effect evaluation test.

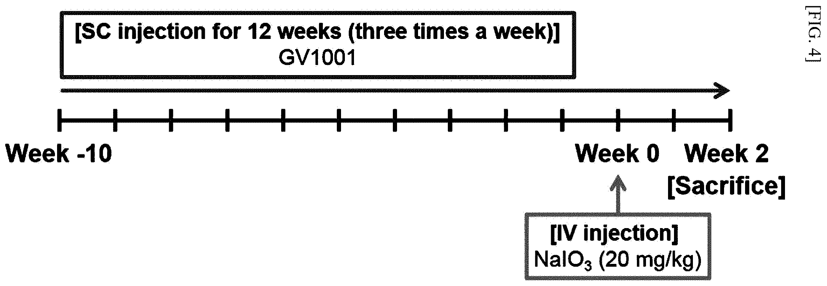

FIG. 4 shows the GV1001 administration time and experimental schedule for the GV1001 pre-treatment animal experimental group used in the GV1001 long-term administration effect evaluation test.

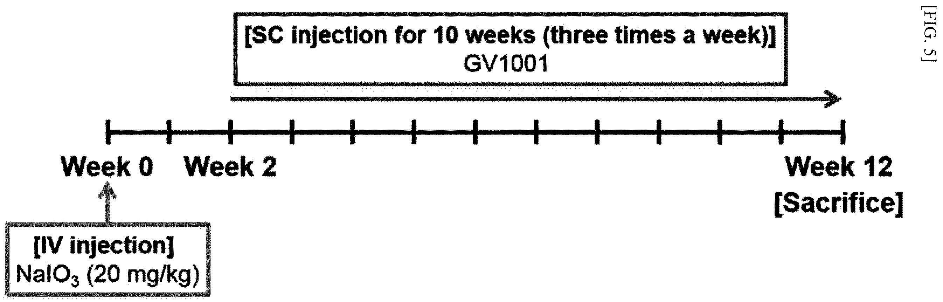

FIG. 5 shows the GV1001 administration time and experimental schedule for the GV1001 post-treatment animal experimental group used in the GV1001 long-term administration effect evaluation test.
FIG. 6 shows the results of confirming the efficacy of GV1001 in reducing reactive oxygen species.
FIG. 7 shows the results of confirming the efficacy of GV 1001 in reducing epithelial-mesenchymal transition.
FIGS. 8 and 9 show the results of confirming the efficacy of short-term GV1001 administration in a mouse model of age-related macular degeneration.
FIGS. 10 and 11 show the results of confirming the efficacy of long-term GV1001 administration in a mouse model of age-related macular degeneration.

### [Mode for Carrying out Invention]

The present invention provides a pharmaceutical composition for preventing or treating macular degeneration, which includes a peptide having the amino acid sequence of SEQ ID NO: 1.

In the present invention, a peptide having the amino acid sequence of SEQ ID NO: 1 includes functional equivalents thereof. The term "functional equivalent" refers to a peptide having at least 70%, 80%, 90%, or 95% sequence identity with the amino acid sequence of SEQ ID NO: 1 as a result of addition, substitution, or deletion of amino acids, and exhibiting substantially the same physiological activity as the peptide having the amino acid sequence of SEQ ID NO: 1. The term "substantially the same physiological activity" refers to an activity involved in the prevention, improvement, or treatment of macular degeneration.

In the present invention, "macular degeneration" encompasses age-related macular degeneration, macular degeneration relevant to age, senile macular degeneration, myopic macular degeneration, and macular dystrophy.

In the present invention, "age-related macular degeneration" includes dry macular degeneration and wet macular degeneration.

In one embodiment, the macular degeneration may be age-related macular degeneration.

In one embodiment, the macular degeneration may be dry macular degeneration.

In the present invention, "prevention" means any act of suppressing or delaying macular degeneration.

In the present invention, "treatment" means any act of improving or beneficially modifying the symptoms of a subject suspected of having, or diagnosed with, macular degeneration.

In one embodiment, the pharmaceutical composition of the present invention may prevent or treat macular degeneration by reducing reactive oxygen species.

In one embodiment, the pharmaceutical composition of the present invention may prevent or treat macular degeneration by reducing degeneration of retinal pigment epithelial cells.

In the present invention, the term "subject" means any animal, including livestock or mice, that has developed or may develop macular degeneration, and may be a mammal including a human.

The pharmaceutical composition of the present invention may include an active ingredient alone, or may be provided as a pharmaceutical composition further including one or more pharmaceutically acceptable carriers, excipients or diluents.

The carrier, excipient or diluent that may be included in the pharmaceutical composition of the present invention may include lactose, dextrose, sucrose, dextrin, maltodextrin, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil, but is not limited thereto.

The present invention provides a kit for preventing or treating macular degeneration, which includes the pharmaceutical composition for preventing or treating macular degeneration and instructions describing a method for preventing or treating macular degeneration.

In one embodiment, a method for preventing or treating macular degeneration may include administering the pharmaceutical composition of the invention to a subject who has developed, or is at risk of developing, macular degeneration.

In the present invention, "administration" means introducing a predetermined substance into a subject by an appropriate method.

The route of administration of the pharmaceutical composition of the present invention may be oral, intravenous, intramuscular, intraarterial, intramedullary, intrathecal, intracardiac, transdermal, subcutaneous, intraperitoneal, intranasal, enteral, topical, sublingual, or rectal administration, but is not limited thereto.

In one embodiment, the composition of the present invention may be administered orally or parenterally.

When the composition of the present invention is administered parenterally, it is preferable to select an administration method such as topical application, intraperitoneal injection, intrarectal injection, subcutaneous injection, intravenous injection, intramuscular injection, or intrathoracic injection, but it is not limited thereto.

The pharmaceutical composition of the present invention may be a solid preparation for oral administration, such as a tablet, pill, powder, granule or capsule.

The pharmaceutical composition of the present invention may be a liquid preparation for oral administration, such as a suspension, a solution, an emulsion or a syrup.

The pharmaceutical composition of the present invention may be a preparation for parenteral administration, such as a sterile aqueous solution, a non-aqueous solution, a suspension, an emulsion, a lyophilized formulation or a suppository.

In one embodiment, a method for preventing or treating macular degeneration may include administering a pharmaceutically effective amount of the pharmaceutical composition of the present invention to a subject who has developed, or is at risk of developing, macular degeneration.

In the present invention, the term "pharmaceutically effective amount" refers to an amount sufficient to treat macular degeneration at a reasonable benefit/risk ratio applicable to medical treatment.

The pharmaceutically effective amount of the pharmaceutical composition of the present invention may be determined according to factors such as the severity of macular degeneration, the activity of the pharmaceutical composition, the sensitivity of the subject or patient to the pharmaceutical composition, the time and route of administration, the excretion rate, the duration of treatment, and concomitant medication, as well as other factors well known in the medical field, and may be appropriately selected by a person skilled in the art.

The pharmaceutical composition of the present invention may be administered as a single therapeutic agent or in combination with other therapeutic agents, may be administered sequentially or simultaneously with conventional therapeutic agents, and may be administered in a single or multiple dosage regimen, which can be readily determined by a person skilled in the art.

The present invention provides a health functional food for preventing or improving macular degeneration, which includes a peptide having the amino acid sequence of SEQ ID NO: 1.

The health functional food of the present invention refers to food manufactured and/or processed in various forms to provide functionality beneficial to the human body.

The health functional food of the present invention may be incorporated into various foods or medicines known in the art.

There is no particular limitation on the type of food in which the health functional food of the present invention may be included. For example, the health functional food of the present invention may be included in meat, sausages, bread, chocolate, candies, snacks, confectionery, pizza, ramen, other noodles, gum, dairy products including ice cream, various soups, beverages, teas, drinks, alcoholic beverages, and vitamin complexes.

The health functional food of the present invention encompasses all forms, including functional foods, nutritional supplements, health foods, and food additives, and may be manufactured in various forms according to conventional methods known in the art. For example, the health food may be manufactured in the form of a liquid drink for oral intake, or may be granulated, encapsulated, tableted into spherical pills, or powdered for consumption. It may also be manufactured in the form of a powder, capsule, soft capsule, tablet, gum, or adhesive-type liquid composition. In addition, the functional food may include beverages (including alcoholic beverages); fruits and processed products thereof (e.g., canned fruits, bottled fruits, jams, marmalades, etc.); fish, meat and processed products thereof (e.g., ham, sausages, corned beef, etc.); breads and noodles (e.g., udon, buckwheat noodles, ramen, spaghetti, macaroni, etc.); fruit juices, various drinks, cookies, taffy, dairy products (e.g., butter, cheese, etc.); edible vegetable oils, margarine, vegetable proteins, retort foods, frozen foods, herbal decoctions, and seasonings (e.g., soybean paste, soy sauce, sauces, etc.).

The health functional food of the present invention may further include ingredients commonly added during food manufacture, as long as it does not deviate from the ultimate purpose of the present invention, and may further include, for example, proteins, carbohydrates, fats, other nutrients, seasonings, and flavoring agents.

The health functional food of the present invention may additionally contain various nutritional supplements, vitamins, electrolytes, flavoring agents, coloring agents, pectic acids and their salts, alginic acid and its salts, organic acids, protective colloid thickeners, pH regulators, stabilizers, preservatives, glycerin, alcohol, carbon dioxide agents used in carbonated beverages, etc.

The health functional food of the present invention may contain fruit pulp for the production of natural fruit juice, fruit juice beverages, and vegetable beverages. These ingredients may be used independently or in combination.

Hereinafter, the present invention will be described in detail by way of examples in order to specifically explain the present invention. However, the following examples are provided only to facilitate understanding of the present invention, and the content of the present invention is not limited thereto.

### Example

Using retinal pigment epithelial cells and a mouse model of age-related macular degeneration, it was confirmed that GV1001 has an effect of reducing damage to the retinal pigment epithelial layer. Further, basic research was conducted to elucidate the molecular mechanism of the pharmacological effects of GV1001 related to macular degeneration.

### 1. Experimental method

### 1.1 Synthesis of GV1001

A peptide consisting of 16 amino acids, having the structure according to Formula 1 below and the amino acid sequence of SEQ ID NO: 1 (GV1001) derived from human telomerase, was synthesized.

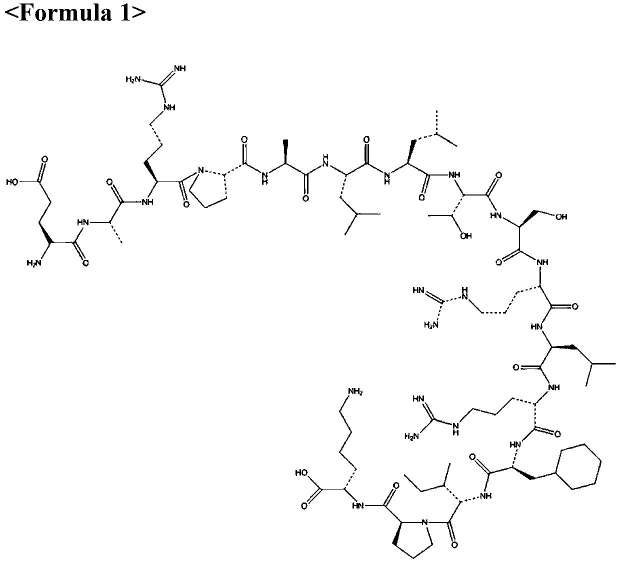

Peptide GV1001 of SEQ ID NO: 1 was prepared according to a conventional solid-phase peptide synthesis method. Specifically, the peptides were synthesized by coupling amino acids sequentially from the C-terminus through Fmoc solid phase peptide synthesis (SPPS) using ASP48S (Peptron, Inc., Daejeon, Korea). The peptides were used, in which the first amino acid at the C-terminus was attached to the resin as follows. For example:
NH₂ -Lys(Boc)-2-chloro-Trityl Resin
NH₂ -Ala-2-chloro-Trityl Resin
NH₂ -Arg(Pbf)-2-chloro-Trityl Resin

All amino acid starting materials used for peptide synthesis were protected at the N-terminus with Fmoc, and all residues were protected with Trt, Boc, t-Bu (t-butylester), Pbf (2,2,4,6,7-pentamethyl dihydro-benzofuran-5-sulfonyl), etc., which are removable under acidic conditions. For example:
Fmoc-Ala-OH, Fmoc-Arg(Pbf)-OH, Fmoc-Glu(OtBu)-OH, Fmoc-Pro-OH, Fmoc-Leu-OH, Fmoc-Ile-OH, Fmoc-Phe-OH, Fmoc-Ser(tBu)-OH, Fmoc-Thr(tBu)-OH, Fmoc-Lys(Boc)-OH, Fmoc-Gln(Trt)-OH, Fmoc-Trp(Boc)-OH, Fmoc-Met-OH, Fmoc-Asn(Trt)-OH, Fmoc-Tyr(tBu)-OH, Fmoc-Ahx-OH, Trt-Mercaptoacetic acid.

As coupling reagents, HBTU [2-(1H-Benzotriazole-1-yl)-1,1,3,3-tetramethylaminium hexafluorophosphate]/ HOBt [N-Hydroxybenzotriazole]/NMM [4-Methylmorpholine] were used. Piperidine in DMF (20%) was used for Fmoc removal. A cleavage cocktail [TFA (trifluoroacetic acid)/TIS (triisopropylsilane)/EDT (ethanedithiol)/H₂O = 92.5/2.5/2.5] was used to cleave the synthesized peptide from the resin and remove protecting groups of the residue.

Using a state in which the starting amino acid having a protecting group bonded thereto was bound to a solid support, a process of reacting each corresponding amino acid, washing with a solvent, and then deprotecting was repeated to synthesize each peptide. The synthesized peptide was cleaved from the resin, purified by HPLC, followed by confirming whether it was synthesized by MS, and then freeze-dried.

The specific synthesis process of GV1001 is described as follows.

### 1) Coupling

NH₂-Lys(Boc)-2-chloro-Trityl resin was added with protected amino acid (8 equivalents) and coupling reagents HBTU (8 equivalents)/HOBt (8 equivalents)/NMM (16 equivalents) dissolved in DMF, followed by reaction at room temperature for 2 hours, and washing with DMF, MeOH, and DMF in this order.

### 2) Fmoc deprotection

Piperidine in DMF (20%) was added and reacted twice for 5 minutes at room temperature, and then washed with DMF, MeOH, and DMF in this order.

3) A peptide backbone was constructed by repeatedly performing reactions 1 and 2.

4) Cleavage: A cleavage cocktail was added to the peptide resin after completion of the synthesis to separate the peptide from the resin.

5) Cooled diethyl ether was added to the obtained mixture, and the resulting peptide was precipitated by centrifugation.

6) After purification by Prep-HPLC, the molecular weight was confirmed by LC/MS, and the product was lyophilized to manufacture powder.

### 1.2 Verification of reduction in retinal pigment epithelial cell degeneration by GV1001

### 1) Establishment of a cell model of age-related macular degeneration

Reactive oxygen species were generated by treating retinal pigment epithelial cell line ARPE-19 with hydrogen peroxide (H₂O₂).

Degenerated retinal pigment epithelial cells that appear in age-related macular degeneration were set as a model cell line, and epithelial loss of retinal pigment epithelial cells was induced.

### 2) Drug dosage and administration

The experimental group for verifying the efficacy of GV1001 in reducing cell degeneration was set up as shown in Table 1 below.

**[TABLE 1]**

| **Group** | **Drug** | |
|---|---|---|
| Negative control | Saline solution | |
| H₂O₂ (50 µM) treatment group | Saline solution | |
| | GV1001 pre-treatment | 0.01 µM |
| | | 0.1 µM |
| | | 1.0 µM |
| | | 10 µM |
| | GV1001 post-treatment | 0.01 µM |
| | | 0.1 µM |
| | | 1.0 µM |
| | | 10 µM |
| | Metformin (1 mM) | |

| | | |
|---|---|---|
| * GV1001 pre-treatment was performed by first treating with GV1001 for 2 hours, followed by H₂O₂ treatment for 24 hours. * GV1001 post-treatment was performed by treating with GV1001 for 2 hours after 24 hours of H₂O₂ treatment. * The positive control drug for GV1001 was metformin (Met), which has been confirmed to have an effect of improving oxidative stress in retinal pigment epithelial cells treated with H₂O₂. Metformin was used at a concentration of 1 mM. * All drugs were diluted in 0.9% saline solution before use. | | |

### 3) Verification of the reduction of retinal pigment epithelial cell degeneration by GV1001

The research details and methods for verifying the efficacy of GV1001 in reducing cell degeneration using H₂O₂-treated retinal pigment epithelial cell line model (ARPE-19) are as shown in Table 2 below.

**[TABLE 2]**

| **Research details** | **Research method** |
|---|---|
| Confirmation of maintenance of epithelial cell properties by GV1001 | The progress of epithelial-mesenchymal transition (EMT), a degeneration process of retinal pigment epithelial cells, was confirmed by observing changes in cadherin 1 (CDH-1) as an epithelial cell marker and vimentin as a mesenchymal cell marker using a confocal microscope. |
| Observation of oxidative stress control by GV1001 | Changes in intercellular reactive oxygen species levels induced by GV1001 were confirmed using a fluorescence microscope with 2',7'-dichlorofluorescin diacetate (DCF-DA) as a reactive oxygen species dye sample. |

### 1.3 Evaluation of the efficacy of short-term GV1001 administration in a mouse model of age-related macular degeneration

### 1) Establishment of an animal model of age-related macular degeneration

Mice injected with sodium iodate (NaIO₃) are used as a representative model of age-related macular degeneration. A mouse model of age-related macular degeneration was established by injecting NaIO₃ into C57BL/6 mice, thereby inducing oxidative stress specific to retinal pigment epithelial cells and causing damage.

### 2) Drug dosage and administration

The animal experimental groups for verifying the efficacy of short-term GV1001 administration in reducing retinal pigment epithelial degeneration were set up as shown in Table 3 below.

**[TABLE 3]**

| **Group** | **Drug** | | **Number of animals** |
|---|---|---|---|
| Negative control | Saline solution | | 3 |
| NaIO₃ treatment group | Saline solution | | 3 |
| | GV1001 pre-treatment | 0.01 mg/kg | 3 |
| | | 0.1 mg/kg | 3 |
| | | 0.5 mg/kg | 3 |
| | | 1 mg/kg | 3 |
| | GV1001 post-treatment | 0.01 mg/kg | 3 |
| | | 0.1 mg/kg | 3 |
| | | 0.5 mg/kg | 3 |
| | | 1 mg/kg | 3 |
| | Metformin (4 mg/mL) | | 3 |

| | | | |
|---|---|---|---|
| * NaIO₃ was injected once at 20 mg/kg via intravenous (IV) injection into the tail vein. * GV1001 was administered 0.01, 0.1, 0.5, or 1.0 mg/kg by subcutaneous (SC) injection three times a week. * For GV1001 pre-treatment, GV1001 was injected subcutaneously three times a week for two weeks starting 4 days before NaIO₃ administration, as shown in FIG. 1. * For GV1001 post-treatment, as shown in FIG. 2, GV1001 was injected subcutaneously three times within one week starting 10 days after NaIO₃ administration. * As a positive control drug for GV1001, metformin 4.0 mg/mL, which has been confirmed to have an improvement effect on retinal pigment epithelial layer degeneration caused by oxidative stress, was used. As shown in FIG. 3, it was diluted in drinking water and administered by replacing it with regular water for 7 days before NaIO₃ administration. * Drugs other than metformin were diluted in saline solution before use. | | | |

### 3) Confirmation of efficacy in reducing damage to retinal pigment epithelium layer by GV1001

To determine whether the disruption of tight junctions in the retinal pigment epithelium caused by NaIO₃ administration was inhibited by GV1001, the retinal pigment epithelium/choroid (RPE/choroid) was flat-mounted, and the junctions between epithelial cells were stained with phalloidin samples and observed using a confocal microscope.

### 1.4 Evaluation of the efficacy of long-term GV1001 administration in a mouse model of age-related macular degeneration

### 1) Establishment of an animal model of age-related macular degeneration

A mouse model of age-related macular degeneration was established by injecting NaIO₃ into C57BL/6 mice and inducing oxidative stress specifically in retinal pigment epithelial cells to cause damage.

### 2) Drug dosage and administration

The GV1001 dose determined through an efficacy evaluation test by short-term administration was used for efficacy evaluation in long-term administration.

The animal experimental group for verification of efficacy in reducing retinal pigment epithelial degeneration by long-term administration (3 months) of GV1001 was set up as shown in Table 4 below.

**[TABLE 4]**

| **Group** | **Drug** | | **Number of animals** |
|---|---|---|---|
| Negative control | Saline solution | | 3 |
| NaIO₃ treatment group | Saline solution | | 3 |
| | GV1001 pre-treatment | 0.1 mg/kg | 3 |
| | | 1.0 mg/kg | 3 |
| Negative control | Saline solution | | 15 |
| NaIO₃ treatment group | Saline solution | | 15 |
| | GV1001 post-treatment | 0.1 mg/kg | 15 |
| | | 1.0 mg/kg | 15 |

| | | | |
|---|---|---|---|
| * NaIO₃ was injected once at 20 mg/kg via intravenous injection into the tail vein. * GV1001 was diluted in saline at 0.1 or 1.0 mg/kg and administered by subcutaneous injection three times a week. * For GV1001 pre-treatment, GV1001 was injected subcutaneously three times a week for 12 weeks starting 10 weeks before NaIO₃ administration, as shown in FIG. 4. * For GV1001 post-treatment, as shown in FIG. 5, GV1001 was injected subcutaneously three times a week for 10 weeks starting 2 weeks after NaIO₃ administration. | | | |

### 3) Confirmation of efficacy in reducing damage to retinal pigment epithelium layer by GV1001

To determine whether the disruption of tight junctions in the retinal pigment epithelium was inhibited by GV1001, the retinal pigment epithelium/choroid was flat-mounted, and the junctions between epithelial cells were stained with phalloidin-stained samples and observed using a confocal microscope to determine the degree of disruption of tight junctions.

### 2. Experimental results

### 2.1 Verification of reduction in retinal pigment epithelial cell degeneration by GV1001

### 1) Reduction of reactive oxygen species by GV1001

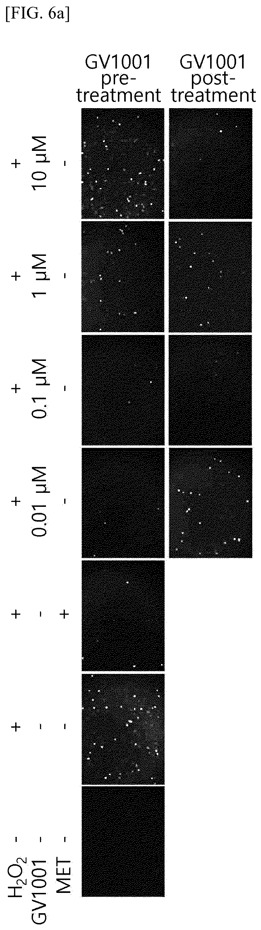

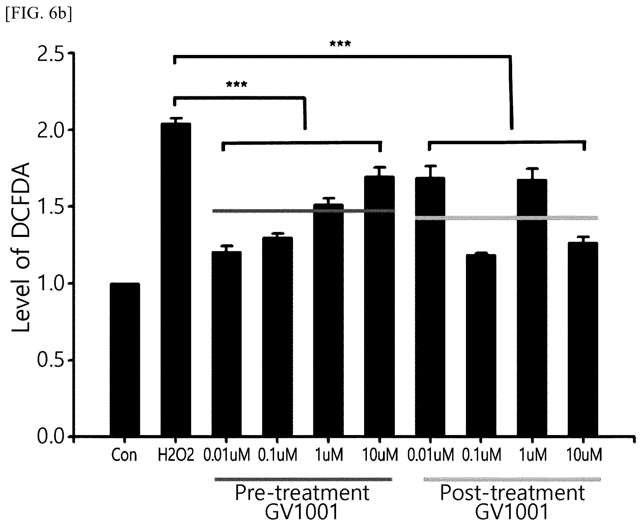

Using DCF-DA as a reactive oxygen species dye sample, the change in the level of reactive oxygen species in retinal pigment epithelial cells (ARPE-19) induced by GV1001 was confirmed by fluorescence microscopy, and the results are shown in FIG. 6. DCF-DA fluorescence intensity increased when oxidative stress was induced by H₂O₂, but decreased by treatment with metformin, a positive control.

Under oxidative stress conditions, DCF-DA fluorescence intensity decreased at a concentration of 0.01 µM in pre-treatment with GV1001, and at concentrations of 0.1 µM and 10 µM in post-treatment.

The increase in reactive oxygen species observed when oxidative stress is applied to retinal pigment epithelial cells may be suppressed or reduced by GV1001. Through this, it was confirmed that GV1001 enables retinal pigment epithelial cells to counteract oxidative stress.

### 2) Reduction of degeneration of retinal pigment epithelial cells by GV1001

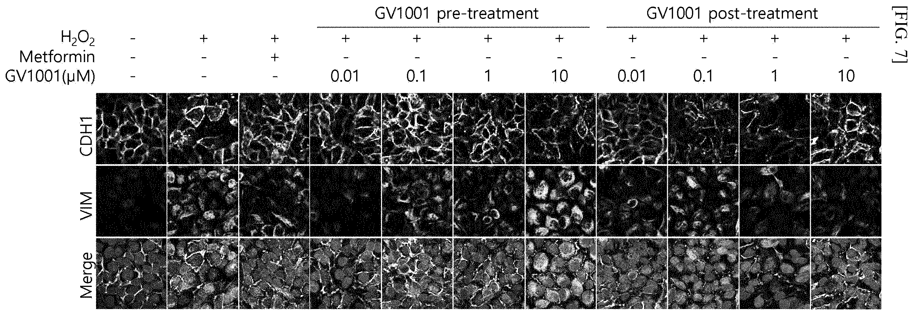

The degree of epithelial-mesenchymal transition, which indicates degeneration of retinal pigment epithelial cells, was confirmed by observing changes in cadherin 1 as an epithelial cell marker, and vimentin as a mesenchymal cell marker, using a confocal microscope, and the results are shown in FIG. 7.

It was confirmed that oxidative stress induced by H₂O₂ decreased the expression of epithelial cell markers and increased the expression of mesenchymal cell markers, thereby promoting epithelial-mesenchymal transition. When treated with metformin as the positive control, the epithelial-mesenchymal transition was reduced.

Epithelial-mesenchymal transition was reduced at a concentration of 0.01 µM in pre-treatment with GV1001, and at a concentration of 10 µM in post-treatment.

The epithelial-mesenchymal transition observed when oxidative stress is applied to retinal pigment epithelial cells may be inhibited or reduced by GV1001. Through this, it was confirmed that GV1001 enables retinal pigment epithelial cells to maintain or restore epithelial properties.

### 2.2 Efficacy evaluation by short-term GV1001 administration in a mouse model of age-related macular degeneration

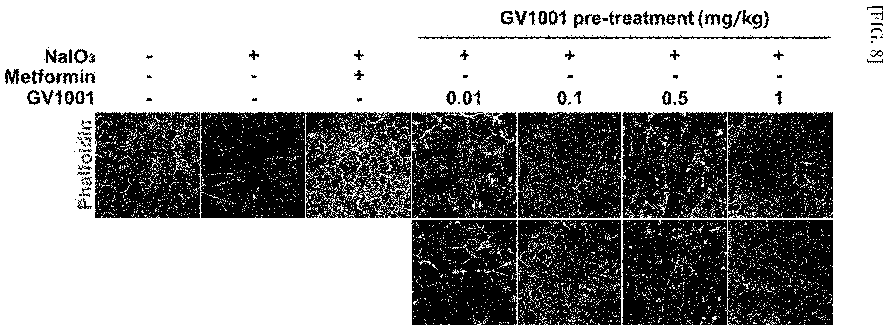

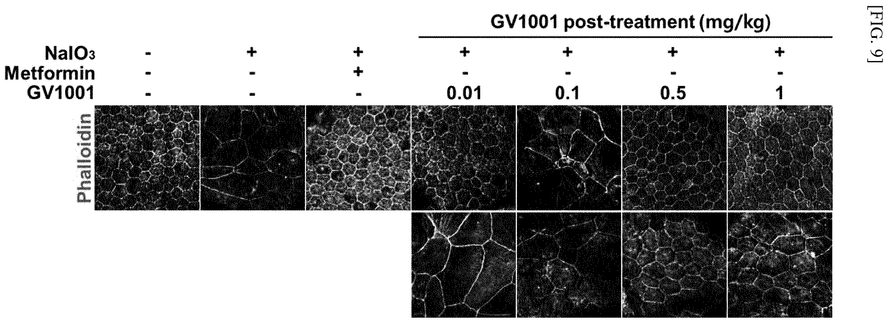

To determine whether the disruption of tight junctions in the retinal pigment epithelial cell layer was inhibited by GV1001, the retinal pigment epithelial layer/choroid was flat-mounted, and the junctions between epithelial cells were stained with phalloidin and observed using a confocal microscope. The results are shown in FIGS. 8 and 9.

In the NaIO₃ treatment group, the tight junction structure of the retinal pigment epithelial cell layer was significantly damaged, indicating a loss of epithelial properties, and was confirmed that the macular degeneration mouse model was well established. When treated with metformin as the positive control group of GV1001, the tight junction structure was maintained.

The tight junction structure of the retinal pigment epithelial cell layer was maintained at 0.1 mg/kg in pre-treatment with GV1001 and at 0.5 and 1.0 mg/kg in post-treatment. This confirmed that GV1001 maintains or restores the epithelial properties of retinal cells in a mouse model of age-related macular degeneration.

### 2.3 Evaluation of the efficacy of long-term GV1001 administration in a mouse model of age-related macular degeneration

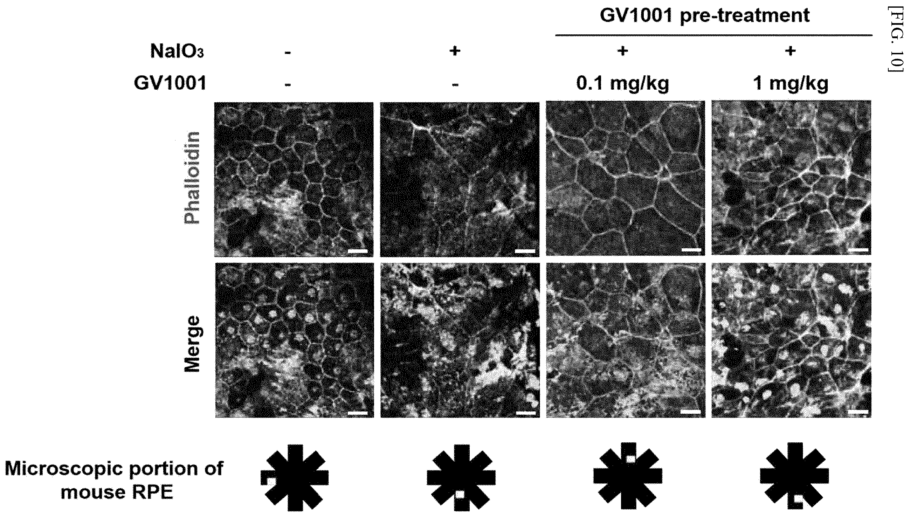

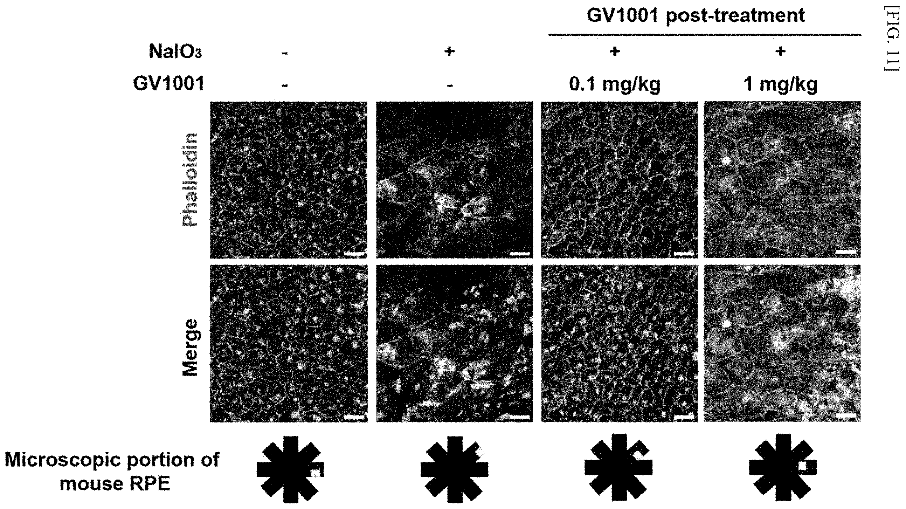

To determine whether the disruption of the tight junctions in the retinal pigment epithelium was reduced by long-term GV1001 administration, the retinal pigment epithelium/choroid was flat-mounted, and the junctions between epithelial cells were stained with phalloidin and observed using a confocal microscope. The results are shown in FIGS. 10 and 11.

In the NaIO₃ treatment group, the tight junction structure of the retinal pigment epithelial cell layer was significantly damaged, indicating a loss of epithelial properties, and it was confirmed that the macular degeneration mouse model was well established.

As shown in FIG. 10, in the case of GV1001 pre-treatment, when GV1001 was administered long-term at 0.1 mg/kg or 1.0 mg/kg, the tight junction structure of the retinal pigment epithelial layer was well maintained, so that cell membrane collapse due to epithelial layer degeneration did not occur, and one nucleus per closed junction cell membrane was observed.

As shown in FIG. 11, in the case of GV1001 post-treatment, when GV1001 was administered long-term at 0.1 mg/kg or 1.0 mg/kg, the tight junction structure of the retinal pigment epithelium was well maintained, so that cell membrane collapse due to epithelial layer degeneration did not occur, and one nucleus per closed junction cell membrane was observed.

### 3. Conclusion

### 3.1 Verification of reduction in retinal pigment epithelial cell degeneration by GV1001

### (1) Reduction of reactive oxygen species by GV1001

The increase in reactive oxygen species observed when oxidative stress is applied to retinal pigment epithelial cells may be inhibited or reduced by GV1001. This means that GV1001 enables retinal pigment epithelial cells to resist oxidative stress.

### (2) Reduction of degeneration of retinal pigment epithelial cells by GV1001

The epithelial-mesenchymal transition observed when oxidative stress is applied to retinal pigment epithelial cells may be inhibited or reduced by GV1001. This means that GV1001 reduces the degeneration of retinal pigment epithelial cells and maintains or restores epithelial properties.

### 3.2 Efficacy evaluation by short-term GV1001 administration in a mouse model of age-related macular degeneration

As a result of pre-treatment or post-treatment with short-term GV1001 in a mouse model of age-related macular degeneration, degeneration of the retinal pigment epithelium was reduced and epithelial properties were maintained or restored. This confirmed the excellent preventive and/or therapeutic efficacy of GV1001 against age-related macular degeneration.

### 3.3 Evaluation of the efficacy of long-term GV1001 administration in a mouse model of age-related macular degeneration

As a result of pre-treatment or post-treatment with long-term GV1001 in a mouse model of age-related macular degeneration, degeneration of the retinal pigment epithelium was reduced and epithelial properties were maintained or restored. This confirmed the excellent preventive and/or therapeutic efficacy of GV1001 against age-related macular degeneration.

## Claims

1. A pharmaceutical composition for use in preventing or treating a macular degeneration, comprising a peptide having the amino acid sequence of SEQ ID NO: 1.

2. The pharmaceutical composition according to claim 1, wherein the macular degeneration is age-related macular degeneration.

3. The pharmaceutical composition according to claim 1, wherein the pharmaceutical composition prevents or treats the macular degeneration by reducing degeneration of retinal pigment epithelial cells.

4. A kit for use in preventing or treating a macular degeneration, comprising:
the pharmaceutical composition according to any one of claims 1 to 3; and
instructions describing a method for preventing or treating the macular degeneration.

5. The kit according to claim 4, wherein the method for preventing or treating the macular degeneration comprises administering the pharmaceutical composition to a subject who has developed, or is at risk of developing, the macular degeneration.

6. A health functional food for use in preventing or improving a macular degeneration, comprising a peptide having the amino acid sequence of SEQ ID NO: 1.

7. The health functional food according to claim 6, wherein the macular degeneration is age-related macular degeneration.

8. The health functional food according to claim 6, wherein the health functional food prevents or improves the macular degeneration by reducing degeneration of retinal pigment epithelial cells.
